# FASCICULE DE BREVET EUROPEEN

(11) **EP 3 334 433 B1**
(45) Date de publication et mention de la délivrance du brevet: **28.09.2022**
(21) Numéro de dépôt: 16760120.2
(22) Date de dépôt: 09.08.2016
(51) Int. Cl.: A61K 31/618, A61K 36/899, A61K 31/35, A61K 36/23, A61K 36/45, A61K 36/53, A61K 36/534, A61K 9/00, A61K 47/08, A61K 47/22, A61K 9/06, A61K 47/32, A61K 47/44, A61K 31/352, A01K 13/00, A01K 27/00, A61D 7/00, A61P 29/00, A61K 47/34, A61P 19/02

(54) **COMPOSITION ANTIDOULEUR COMPRENANT UN DERIVE DE L'ACIDE SALICYLIQUE**
SCHMERZBETÄUBENDE ZUSAMMENSETZUNG MIT EINEM SALICYLSÄUREDERIVAT
PAIN-KILLING COMPOSITION COMPRISING A SALICYLIC ACID DERIVATIVE

(30) Priorité: 12.08.2015 FR 1501724
(43) Date de publication de la demande: 20.06.2018
(73) Titulaire: AB7 Santé, 31450 Deyme (FR)
(72) Inventeur: CHELLE, René, 31450 Deyme (FR)
(86) Numéro de dépôt international: PCT/FR2016/000129
(87) Numéro de publication internationale: WO 2017/025666

(56) Documents cités:
- EP-A1- 2 047 845
- AU-A- 3 723 589
- CA-A1- 2 383 806
- CN-A- 101 757 117
- CN-A- 104 491 767
- FR-A1- 2 901 132
- US-B1- 8 865 234
- US-B2- 8 802 736
- Anonymous: "Uniquely Emu Products, Inc.: PRO80 For Sore Muscles & Joints 2 oz", , 7 mai 2015 (2015-05-07), XP055320688, Extrait de l'Internet: URL:https://web.archive.org/web/2015050714 3807/http://www.uniquelyemu.com/UE2002/PRO 80-For-Sore-Muscles--Joints-2-oz.htm [extrait le 2016-11-17]
- Anonymous: "Wintergreen DL Brochure", , 11 septembre 2014 (2014-09-11), XP055320690, Extrait de l'Internet: URL:http://wintergreen.co.za/wp-content/up loads/2014/09/Wintergreen-DL-Brochure_Web- download.pdf [extrait le 2016-11-17]
- DATABASE WPI Week 201058 Thomson Scientific, London, GB; AN 2010-J98379 XP002764386, -& CN 101 757 117 A (FAANG H) 30 juin 2010 (2010-06-30)
- DATABASE WPI Week 201566 Thomson Scientific, London, GB; AN 2015-341958 XP002764387, -& CN 104 491 767 A (LI X) 8 avril 2015 (2015-04-08)
- NAWAZ A ET AL: "Clinical efficacy of polyherbal formulation Eezpain spray for muscular pain relief", PAKISTAN JOURNAL OF PHARMACEUTICAL SCIENCES, FACULTY OF PHARMACY, UNIVERSITY OF KARACHI, PK , vol. 28, no. 1 1 janvier 2015 (2015-01-01), pages 43-47, XP008182313, ISSN: 1011-601X Extrait de l'Internet: URL:http://www.pjps.pk/wp-content/uploads/ pdfs/28/1/Paper-7.pdf
- MEHTA N J ET AL: "Development and evaluation of antiarthritic herbal ointment", RESEARCH JOURNAL OF PHARMACEUTICAL, BIOLOGICAL AND CHEMICAL SCIENCES, RESEARCH JOURNAL OF PHARMACEUTICAL, BIOLOGICAL AND CHEMICAL SCIENCES, IN , vol. 4, no. 1 1 janvier 2013 (2013-01-01), pages 221-228, XP008182314, ISSN: 0975-8585 Extrait de l'Internet: URL:http://www.rjpbcs.com/pdf/2013_4%281%2 9/[25].pdf
- HIGASHI Y ET AL: "Efficacy and safety profile of a topical methyl salicylate and menthol patch in adult patients with mild to moderate muscle strain: A randomized, double-blind, parallel-group, placebo-controlled, multicenter study", CLINICAL THERAPEUTICS, EXCERPTA MEDICA, PRINCETON, NJ, US, vol. 32, no. 1, 1 janvier 2010 (2010-01-01), pages 34-43, XP026908522, ISSN: 0149-2918, DOI: 10.1016/J.CLINTHERA.2010.01.016 [extrait le 2010-02-08]
- ANNA HERMAN ET AL: "Essential oils and their constituents as skin penetration enhancer for transdermal drug delivery: a review", JOURNAL OF PHARMACY AND PHARMACOLOGY, vol. 67, no. 4, 31 décembre 2014 (2014-12-31), pages 473-485, XP055282508, LONDON; GB ISSN: 0022-3573, DOI: 10.1111/jphp.12334
- BHARTI SAPRA ET AL: "Percutaneous Permeation Enhancement by Terpenes: Mechanistic View", THE AAPS JOURNAL, vol. 10, no. 1, 8 février 2008 (2008-02-08), pages 120-132, XP055273493, DOI: 10.1208/s12248-008-9012-0
- AQIL ET AL: "Status of terpenes as skin penetration enhancers", DRUG DISCOVERY TODAY, ELSEVIER, RAHWAY, NJ, US, vol. 12, no. 23-24, 17 octobre 2007 (2007-10-17), pages 1061-1067, XP022370274, ISSN: 1359-6446, DOI: 10.1016/J.DRUDIS.2007.09.001

## Description

La présente invention se situe dans le domaine des dispositifs à action thérapeutique comprenant un agent actif destiné à induire une action systémique chez une cible. Plus précisément, la présente invention porte sur un dispositif monobloc à action thérapeutique, à base d'une matrice polymère contenant un agent actif constitué d'au moins un dérivé de l'acide salicylique destinée à une application topique pour un soulagement de douleurs musculaires et/ou articulaires d'un sujet, comme défini dans les revendications.

Comme pour tous les êtres vivants, le phénomène de vieillissement des tissus est doublé d'une baisse du tonus musculaire. Cette baisse peut parfois s'accompagner d'autres inconforts tels que des douleurs et picotements ressentis aux différents endroits du corps notamment au niveau des muscles, des articulations et des tendons.

L'arthrose est une affection articulaire se traduisant par la dégénérescence du cartilage. Le cartilage, en se déshydratant perd son élasticité et sa souplesse, s'amincit et peut parfois disparaître. L'arthrose se manifeste par des douleurs qui s'accentuent au froid. Elle se traduit également par la raideur des membres et réduit ainsi la motricité du sujet qui en est atteint. La situation est encore plus inconfortable lorsque des problèmes musculaires se combinent avec l'arthrose, pénalisant davantage les sujets atteints par rapport à leur mobilité, leur motricité pour effectuer des gestes simples du quotidien.

Il existe de nombreux principes actifs, tant d'origine naturelle que de synthèse, utilisés pour remédier à ces inconforts lorsque le sujet éprouve des sensations de douleur allant de faible à aigue au niveau des muscles et des articulations. Dans ce domaine, l'acide salicylique et ses dérivés sont des principes actifs anti-inflammatoires communément usités et également reconnus pour leur efficacité.

Depuis des siècles, on connaît l'utilisation des huiles essentielles pour leurs nombreuses propriétés à savoir notamment antalgiques, anti-inflammatoires, drainantes, relaxantes, décontractantes, antibactériennes et antifongiques. Pour la facilité d'utilisation, les huiles essentielles sont fréquemment formulées soit en semi-solide (pommades, crèmes, gels) soit en liquide (laits, émulsions, lotions). De plus, des agents de perméation sont systématiquement ajoutés dans ces formulations. De fait, elles sont destinées à une application topique, qui pour produire les effets systémiques escomptés, nécessite des applications répétées et contraignantes dans la journée (Morra P. et *al.,* 1996 ; 30 : 935-940).

Plusieurs facteurs influencent l'absorption cutanée à savoir la formulation de la substance active, son mode d'application sur la peau, qui tient compte de la surface de peau exposée, de la dose par unité de surface, de la durée de contact et des applications multiples. Il a été établi depuis longtemps qu'une substance, au lieu de passer entièrement à travers la peau, peut rester en partie sur la peau et peut constituer un réservoir et être libérée, ou non, ultérieurement. Cet effet est notamment observé avec le salicylate de méthyle lorsque ce dernier fait l'objet d'une application topique. En effet, lors d'une application topique de ces formulations, non seulement, une grande proportion du salicylate de méthyle reste à la surface de la peau mais également qu'un temps d'environ 60 minutes doit être observé avant qu'il ne soit détectable dans le système circulatoire (Sheree E. Cross et *al.,* 1998 ; 46 : 29-35) afin d'induire une action systémique.

Dans les véhicules d'application topique, les huiles essentielles sont dispersées dans au moins une huile végétale laquelle joue un double rôle, à savoir le rôle de dispersant et celui d'émollient pour promouvoir le passage transdermique des molécules actives des huiles essentielles.

Dans le domaine humain et animal, pour les traitements systémiques au moyen d'un véhicule d'application topique solide libérant l'agent actif, l'état de la technique antérieure divulgue au moins deux types de systèmes. D'une part, les systèmes à réservoir constitués par au moins une membrane perméable audit agent actif et un réservoir dans lequel est stocké ledit agent formulé soit en liquide soit en gel. D'autre part, les systèmes matriciels multicouches dont au moins une couche, celle en contact avec la peau, est adhésive.

De cette manière, on évite, certes, les applications répétées mais on ne s'affranchit pas forcément de certains désagréments tels que les irritations et les allergies provoquées par certains solvants hydrophiles tels que les alcools sans parler du bouchage des pores liés au port prolongé desdits dispositifs.

Des agents promoteurs de perméation spécifiques sont fréquemment associés aux deux systèmes susmentionnés afin de promouvoir le passage transdermique de l'agent actif à travers la peau, plus précisément pour franchir le stratum corneum et induire ainsi une action systémique. De tels agents de perméation sont ceux classiquement connus de l'Homme du métier à savoir notamment les acides gras, les esters d'acides gras comprenant 10 à 20 atomes de carbone, les ester-lactates d'acides gras, les monoglycérides et leurs dérivés, les acyl-lactates, et les alcools inférieurs comprenant de 2 à 4 atomes de carbone.

Le brevet FR2901172 décrit un procédé d'incorporation d'un liquide, notamment des huiles essentielles, dans des granulés de polymères tels qu'un copolymère d'éthylène et d'acétate de vinyle et un polyéther block amide. Mais, ce brevet ne suggère aucunement d'utiliser cette technique pour l'obtention d'une composition comprenant un agent actif comprenant au moins un dérivé de l'acide salicylique pour soulager les douleurs musculaires et/ou articulaires d'un sujet.

Le brevet FR2901132 divulgue un dispositif à application transdermique chargé d'agents actifs choisis parmi des huiles essentielles ainsi que des molécules de synthèse solubles dans une huile végétale.

Mais, ce document n'envisage aucunement une composition comprenant un agent actif constitué d'au moins un dérivé de l'acide salicylique et d'une huile essentielle pour un soulagement des douleurs articulaires et/ou musculaires d'un sujet.

Il a été démontré que lorsque le salicylate de méthyle est employé comme principe actif dans une composition, une grande proportion de ce composé ne parvient pas franchir l'épiderme lorsqu'une formulation en crème, en gel ou liquide le contenant est appliquée par voie topique (Sheree E. Cross et *al,* 1998, 46 : 29-35). Ce phénomène s'explique notamment par le faible taux d'hydrolyse du dérivé de l'acide salicylique par les estérases de la peau. La vitesse de passage transdermique du salicylate de méthyle est donc réduite. Il est connu que le stratum corneum ou couche cornée constitue une barrière étanche si bien que de nombreuses molécules ne peuvent la franchir pour aller jusque dans le système circulatoire. Par conséquent, l'utilisation des agents de perméation est l'une des approches fiables permettant de faciliter le passage transépidermique ; mais d'une manière générale, ces agents interagissent avec certains des composants du stratum corneum pour augmenter la pénétration des molécules actives pour induire une action systémique (Charoo Nasseem et *al.,* 2005, 10 : 343-351) ce qui constitue un inconvénient.

Du fait que le dérivé de l'acide salicylique a une vitesse de passage transcutané réduite, les compositions topiques le contenant sont à appliquer en plusieurs fois. Cela cause donc un problème d'accumulation du dérivé de l'acide salicylique, notamment des sels de salicylate à la surface de la peau (Neubert R. et *al,* 1990 ; 176 : 711-716).

On connaît par le brevet canadien n°2383806 une composition contenant du salicylate de méthyle, de l'huile de coco, d'une huile essentielle pour traiter les douleurs par application par voie topique. Dans la composition, le salicylate de méthyle est considéré, soit comme agent de dilatation, soit comme principe actif, et est mélangé avec un alcool cétylique. Les huiles essentielles de romarin, de camphre, de bouleau, de laurier et de sauge peuvent être présentes dans la composition. Le brevet australien n°3723589 décrit une composition anti-inflammatoire comprenant un sel de salicylate de cuivre, du salicylate de méthyle ainsi que d'autres huiles essentielles telles que l'eucalyptus, la menthe et le romarin. On connaît du brevet américain n°8802736 une composition anti-inflammatoire comprenant un agoniste sélectif TRPV1 (capsaïcine et ses dérivés) et du salicylate de méthyle en tant que solvant capable de solubiliser ledit agoniste. L'objectif poursuivi par ce brevet est d'augmenter la vitesse de perméation de la capsaïcine. On connaît par document DE102010053939 une composition pour le traitement de douleurs musculaires comportant de l'héparine, du menthol, d'hydroxyéthylesalicylate, du Diclofenac et d'huile essentielle de romarin dont la fonction est de stimuler la circulation sanguine. Le brevet européen EP2047845 décrit une préparation adhésive anti-inflammatoire comportant du salicylate de méthyle et du 1-menthol où ce dernier a pour fonction de limiter l'évaporation du salicylate de méthyle, et d'agent de promotion de l'absorption percutanée du salicylate de méthyle lequel est présent à raison de 3 à 60 ng.heure/mL dans le plasma sanguin pendant une période allant de 0 à 24 heures ; l'humidité relative du patch étant de 90%. On connaît par le document publié le 31 décembre 2014, intitulé *« Essential oils and their constituents as skin pénétration enhancerfor transdermal drug delivery: a review »* écrit par Anna Herman et al., des mécanismes d'action impliquant des huiles essentielles et leurs constituants majeurs en tant qu'agents de perméation de la peau pour l'administration transdermique de médicaments. Le salicylate de méthyle est absent de la liste des principes actifs. On connaît par le document publié le 8 février 2008, intitulé *« Percutanous permeation enhancement by terpenes : mechanistic view »* écrit par Bharti Sapra et al., des mécanismes selon lesquels les terpènes augmentent la perméation transdermique de médicaments à savoir l'interaction des médicaments avec la couche lipidique du stratum corneum (SC) ou l'augmentation de leur solubilité vis-à-vis des lipides du SC. Le 1-8 cinéole améliore le passage transcutané du 5-fluororacil, la Zidovudine, de l'acide méfénamique ; le menthol celui de la Nicardipine hydrochloride, de la Zidovudine ; le linalol celui de l'Haloperidol et de la Zidovudine. Le salicylate de méthyle est absent de la liste des principes actifs. On connaît par document publié le 17 octobre 2007, intitulé *« Status of terpenes as skin pénétration enhancers »* écrit par Aqil Mohammed et al., des applications de terpènes en tant que promoteurs de perméation de médicaments à travers la peau. Le menthol et le géraniol y sont cités en tants d'agents de perméation de médicaments hydrophiles tels que la caféine, l'hydrochloride d'imipramine ; le linalol et le 1-8 cinéole pour l'hydrochloride de propanolol. Le salicylate de méthyle est absent de la liste des principes actifs. Mais, aucun des documents de l'art antérieur ne divulgue l'utilisation d'une composition comportant du salicylate de méthyle dont la vitesse de transport à travers la peau est augmentée.

Il existe donc un besoin de fournir un dispositif contenant une composition comprenant un agent actif comportant un dérivé de l'acide salicylique, à savoir du salicylate de méthyle dont la vitesse de pénétration cutanée serait plus rapide, ledit agent actif étant capable d'apporter une action systémique pour un soulagement satisfaisant des douleurs musculaires et/ou articulaires, voire les tendinites, d'un sujet animal. Il existe également un besoin d'augmenter l'action thérapeutique de soulagement de douleurs par augmentation de la vitesse de transport du salicylate de méthyle à travers la peau.

La présente invention se propose de surmonter les inconvénients susmentionnés en fournissant un dispositif monobloc à action thérapeutique contenant une composition comprenant un agent actif constitué d'au moins un dérivé de l'acide salicylique et au moins une huile essentielle auxiliaire dans un véhicule d'application topique afin d'induire une action systémique chez un sujet pour un soulagement des douleurs musculaires et/ou articulaires, comme défini dans les revendications.

En particulier, la présente invention porte sur un dispositif monobloc à action thérapeutique, qui comporte un véhicule d'application topique en matrice polymère et un agent actif induisant un soulagement de douleurs musculaires et/ou articulaires d'un sujet humain ou animal, ledit agent actif comportant du salicylate de méthyle apporté par l'huile essentielle de gaulthérie couchée et au moins une huile essentielle auxiliaire, ledit dispositif étant un collier, un bracelet ou un patch,caractérisé en ce que l'agent actif est incorporé dans ladite matrice polymère, qui représente de 80 à 90% en poids du dispositif, et que ledit agent actif comporte 52 à 85% en poids de salicylate de méthyle, la vitesse de transport du salicylate de méthyle à travers la peau étant augmentée grâce à au moins une huile essentielle auxiliaire choisi parmi l'huile essentielle de romarin officinal ou l'huile essentielle de citronnelle de Ceylan.

La présente invention porte en outre sur un dispositif monobloc à action thérapeutique, qui comporte un véhicule d'application topique en matrice polymère et un agent actif induisant un soulagement de douleurs musculaires et/ ou articulaires d'un sujet humain ou animal, ledit agent actif comportant du salicylate de méthyle apporté par l'huile essentielle de gaulthérie couchée et au moins une huile essentielle auxiliaire, ledit dispositif étant un collier ou un bracelet, caractérisé en ce que l'agent actif est incorporé dans ladite matrice polymère, qui représente de 80 à 90% en poids du dispositif, et que ledit agent actif comporte 52 à 85% en poids de salicylate de méthyle, la vitesse de transport du salicylate de méthyle à travers la peau étant augmentée grâce à l'huile essentielle auxiliaire de menthe poivrée.

Ainsi, un premier objet de la présente invention est un dispositif monobloc à action thérapeutique comportant, dans un véhicule d'application, au moins une huile essentielle pour induire un soulagement de douleurs musculaires et/ou articulaires d'un sujet animal, ladite composition traitant par voie topique une zone de la peau dudit sujet, caractérisée en ce que ladite composition comporte du salicylate de méthyle, qui constitue, avec au moins une huile essentielle de la composition n'en contenant que peu ou pas du tout, un agent actif au sein duquel la vitesse de transport du salicylate de méthyle à travers la peau est augmentée grâce à l'huile essentielle, comme défini dans les revendications.

Dans un mode d'utilisation de l'invention, la composition comporte un agent actif comprenant un salicylate de méthyle et au moins une huile essentielle auxiliaire, ledit agent actif étant compris dans un véhicule d'application topique comme défini dans les revendications, pour soulager les douleurs musculaires et/ou articulaires et ainsi améliorer la vitalité d'un sujet animal. Le salicylate de méthyle est avantageusement apporté à la composition par l'huile essentielle de gaulthérie couchée ; sa quantité totale dans la composition est telle qu'elle permet de soulager les douleurs musculaires et/ou articulaires d'un sujet animal par une action systémique.

Avantageusement, l'huile essentielle de gaulthérie couchée constitue de 1 à 15% en poids et la (ou les) huile(s) essentielle(s) auxiliaire(s) mise(s) en œuvre constitue(nt) de 0,15 à 9% en poids par rapport au poids total de l'ensemble constitué de toutes les huiles essentielles de la composition incorporée dans son véhicule d'application topique pour la mise en œuvre dans l'utilisation. Le salicylate de méthyle constitue de 52 à 85% en poids du poids total de l'agent actif. L'huile essentielle auxiliaire peut constituer entre 15 et 48% en poids du poids total de l'agent actif. Dans ces conditions, le soulagement des douleurs peut intervenir sans accumulation du salicylate de méthyle sur la zone de peau à traiter.

Au sens de la présente invention, on entend par « vitalité » l'état physique du sujet qui se traduit par un soulagement des douleurs musculaires et/ou articulaires et par un regain de la tonicité musculaire du fait de la décongestion veineuse et lymphatique.

On entend par « sujet animal » la cible lorsqu'il s'agit de l'Homme ou d'un animal, notamment un animal de compagnie tel qu'-un chien, un chat ou un cheval.

On entend par « huile essentielle auxiliaire » une huile essentielle ou un mélange d'huiles essentielles extraite(s) d'une plante dont la fonction est d'entraîner le salicylate de méthyle à passer à travers la peau, ladite huile essentielle ne contenant que peu de salicylate de méthyle c'est-à-dire que ce dernier s'y retrouve en faible quantité, moins de 1%.

De manière inattendue, l'inventeur a constaté que, lorsqu'un agent actif est constitué d'un dérivé de l'acide salicylique, notamment le salicylate de méthyle, associé à au moins une huile essentielle, le salicylate de méthyle est entrainé par une huile essentielle dite « auxiliaire » de sorte que l'ensemble passe la barrière cutanée sur la zone de peau à traiter. L'huile essentielle auxiliaire permet d'augmenter la vitesse de pénétration transdermique et de transport à travers la peau d'un dérivé de l'acide salicylique, donc du salicylate de méthyle. De cette manière, d'une part, le dérivé de l'acide salicylique, en l'occurrence le salicylate de méthyle, ne s'accumule plus sur la surface de la zone de peau à traiter, et d'autre part, il n'est plus nécessaire de procéder à des applications topiques répétées d'une composition comprenant ledit agent actif pour obtenir un effet de soulagement des douleurs articulaires et/ou musculaires du sujet traité.

Le dérivé de l'acide salicylique de la présente invention est le salicylate de méthyle. Le salicylate de méthyle est apporté à la composition par l'huile essentielle de gaulthérie couchée (*Gaultheria procumbens* et *Gaultheria fragrantissima*)*,* qui peut en contenir jusqu'à 100% en poids. Le dispositif monobloc contient au moins une huile essentielle auxiliaire contenant avantageusement au moins un alcool monoterpénique. Les alcools monoterpéniques sont pris de préférence dans le groupe formé par le 1-8-cinéole, le menthol, le géraniol, le linalol ou un mélange de ceux-ci ; lesdits alcools ont la propriété de pouvoir passer spontanément la barrière cutanée. Au surplus, l'huile essentielle auxiliaire, outre sa propriété susmentionnée, apporte également des effets bénéfiques pour le sujet traité tels que la décontraction musculaire, la détente musculaire ou encore la vasodilatation.

Selon un mode d'utilisation du dispositif monobloc de l'invention, elle met en œuvre l'huile essentielle auxiliaire qui est avantageusement l'huile essentielle de romarin officinal (*Rosmarinus officinalis*)*,* laquelle contient du 1-8-cinéole.

Selon un autre mode d'utilisation du dispositif monobloc de l'invention, comme huile essentielle, elle met en œuvre l'huile essentielle auxiliaire comportant l'huile essentielle de menthe poivrée (*Mentha piperita*)*,* laquelle contient du menthol et/ou l'huile essentielle de Ceylan (*Cymbopogon nardus*)*,* laquelle contient du géraniol et/ou l'huile essentielle de coriandre (*Coriandrum sativum*)*,* laquelle contient du linalol et ne fait pas partie de l'objet de l'invention.

La quantité totale des alcools monoterpéniques dans la composition ne doit pas provoquer de réaction allergique ou toxique chez le sujet traité.

La composition comporte également un véhicule d'application topique comme défini dans les revendications.

Selon un mode d'utilisation ne faisant pas partie de l'objet de l'invention, ledit véhicule d'application topique est un fluide pris dans le groupe formé par un liquide dont la viscosité permet une distribution en aérosol, un shampooing, une lotion, un lait ou une émulsion.

Selon un autre mode d'utilisation ne faisant pas partie de l'objet de l'invention, le véhicule d'application topique peut prendre la forme d'un gel, d'une pommade ou d'une crème.

Selon le mode d'utilisation du dispositif monobloc de l'invention, le véhicule d'application topique est une matrice polymère dans laquelle est incorporé l'agent actif.

Dans une première variante, ladite matrice polymère est formée à partir d'un copolymère d'éthylène et d'acétate de vinyle (EVA), dont le taux d'acétate de vinyle est compris entre 15 et 60% en poids.

Dans une deuxième variante de ce mode d'utilisation du dispositif monobloc de l'invention, la matrice polymère est un polymère thermoplastique choisis dans le groupe formé par les élastomères de polyuréthane thermoplastiques à base d'esters ou d'éthers, les copolyamides et les polyamides (PA) de grades absorbants, tels que PA6, PA10, PA12, les polyéthers block amide de grades absorbants, les polyéthylènes de grades absorbants, les polyéthylènes greffés amidon, les polyesters, les polymères styréniques tels que les SEBS, SIS, les polyoléfines, les polychlorures de vinyle ou un mélange de ceux-ci, l'agent actif étant incorporé dans ladite matrice.

Dans une troisième variante de ce mode d'utilisation du dispositif monobloc de l'invention, la matrice polymère est un polyuréthane réticulé à base de d'isocyanate et de polyols ou une polyurée à base d'isocyanate et de polyamine. Le polyuréthane réticulé est obtenu à partir d'une résine polyol liquide ou rendue liquide et un isocyanate liquide ou rendu liquide et une puis mis en forme par coulée. La polyurée réticulée est obtenue à partir d'une résine polyamine liquide ou rendue liquide et un isocyanate liquide ou rendu liquide et une puis mis en forme par coulée. L'isocyanate est choisi parmi le groupe formé par les isocyanates avec au minimum 2 fonctionnalités, de structure aromatique ou aliphatique. On peut citer les TDI (diisocyanate de toluène), HDI (diisocyanate d'hexaméthylène), MDI (diisocyanate de diphénylméthylène), H12MDI (dicyclohéxylméthane diisocyanate), IPDI (isophorone diisocyanate), NDI (naphtalène diisocyanate), TODI (O-tolidine diisocyanate), PPDI (para-phénylène diisocyanate) et leurs prépolymères.

La résine polyol est choisie parmi celle ayant au minimum 2 fonctionnalités hydroxyle, à chaîne longue ou à chaîne courte, à base de polyesters, de polyéthers, de polythioéthers, de polyacétals, de polycarbonates, de polyesteramides, d'huiles végétales naturellement hydroxylées ou modifiées ou le mélange de ceux-ci. Les polyamines sont celles ayant au moins 2 fonctionnalités amine, choisies parmi les polyétheramines, aliphatiques ou aromatiques.

Le procédé d'incorporation d'au moins un agent actif liquide dans le polymère de polyuréthane réticulé susmentionné se fait selon les enseignements du brevet FR2992325. Le procédé consiste à mélanger, à la température ambiante, la composition liquide avec la phase polyol liquide jusqu'à obtention d'un mélange homogène. Parallèlement, on prépare la phase isocyanate liquide. Ensuite, on mélange les deux phases par agitation pendant environ 30 secondes puis on coule le mélange liquide homogène obtenu dans un moule pour former la matrice.

Le véhicule d'application topique est une matrice polymère, qui se présente sous forme d'un dispositif « monobloc », c'est à dire qu'il est dépourvu à la fois de membrane de régulation contrôlant l'évaporation du dérivé d'acide salicylique incorporé et de couche adhésive pour promouvoir le contact avec la peau. Ladite matrice polymère peut être mise en forme de dispositifs comme les colliers, les bracelets, patchs et analogues que l'on maintient contre la peau au moyen, par exemple, d'une orthèse.

Dans le cas d'un véhicule d'application topique en matrice polymère thermoplastique, ladite matrice est mise en forme par les techniques de plasturgie bien connues de l'Homme du métier à savoir par extrusion, par injection moulage ou encore par pressage. Le mode d'incorporation de la composition dans le polymère peut être réalisé selon les enseignements du brevet FR2901172. Ce procédé permet d'incorporer à froid un liquide contenant une ou plusieurs substances actives dans un polymère, à une température voisine de 1 à 5°C au dessus de sa température de transition vitreuse.

Le véhicule d'application topique est une matrice polymère, et représente entre 80 et 90% en poids par rapport au poids total de la composition. Si la matrice est un copolymère EVA, elle est de préférence, formée à partir des granulés ou de poudre de copolymères EVA dont le taux d'acétate de vinyle est compris entre 15% et 60% en poids.

Selon un mode d'utilisation du dispositif monobloc de l'invention, la composition contient au moins un additif de formulation choisi selon le véhicule d'application topique utilisé. L'additif de formulation peut être un vecteur permettant de favoriser le relargage de l'agent actif à partir du véhicule d'application topique ou bien des agents participant à la structure et/ou à la mise en forme dudit véhicule. Les additifs de formulation pouvant être choisis parmi l'eau, des huiles végétales, des solvants miscibles à l'eau, des charges inertes d'origine minérale sous forme de poudre, des agents gélifiants, des colorants, des tensioactifs, du gaz propulseur, des agents épaississants, du parfum de synthèse ou d'origine naturelle ou un mélange de ceux-ci.

L'ensemble des additifs de formulation et le véhicule d'application peut représenter jusqu'à 98% en poids du poids total de la composition mise en œuvre dans l'utilisation.

Les quantités effectives de chacun des constituants de l'agent actif sont calculées de sorte que l'on évite, d'une part, l'accumulation du salicylate de méthyle sur la zone de peau à traiter et d'autre part induire une action systémique relativement rapide.

L'huile végétale est choisie parmi l'huile de coprah raffinée, de macadamia raffinée, d'onagre, de citron, d'amande douce, de tamanu ou le mélange de celles-ci. L'huile végétale permet d'améliorer la miscibilité de l'huile essentielle de gaulthérie et de l'huile essentielle auxiliaire entre elles. Elle permet également de diluer la concentration du mélange d'huiles essentielles au sein de la composition liquide. Accessoirement, l'huile végétale joue le rôle de dispersant des huiles essentielles. Lorsque l'huile végétale est présente, sa quantité varie entre 5 et 15% en poids par rapport au poids total de l'agent actif.

Les charges minérales sont choisies dans le groupe formé par le talc, les oxydes de zinc ou de titane, les micas, la silice, le carbonate de calcium, les particules d'argile, le liège ou un mélange de celles-ci. Dans le cas où le véhicule d'application est une matrice polymère, lesdites charges ont pour fonction d'améliorer la tenue mécanique de la composition et aussi de booster le relargage de l'agent actif. En effet, lorsque la quantité totale desdites charges dans la composition est importante, la vitesse de relargage de l'agent actif s'en trouve accélérée. Lorsque les charges sont présentes dans la composition, elles sont préférentiellement mélangées directement avec le polymère en une quantité variant entre 0,1 et 5% en poids de la composition.

Les colorants sont ceux couramment utilisés et connus de l'homme du métier. Ils peuvent être sous forme solide en granulés ou en poudre, ou sous forme liquide. Lorsque le colorant est présent, il est mélangé directement avec le véhicule d'application topique utilisé.

Le parfum peut être d'origine naturelle ou de synthèse dont la quantité peut représenter entre 0 et 15% en poids de la composition mise en œuvre dans l'utilisation. Il peut être apporté directement par l'une des huiles essentielles de la composition. Le parfum est, par exemple, choisi parmi le parfum de vanille, de citron vert, de lavande, de violette, de pomme, d'abricot, de patchouly, de bambou feuilles vertes et peut être adapté en fonction du goût de l'usager final.

Les agents épaississants sont ceux couramment utilisés en cosmétique dans les crèmes. Ils sont choisis dans le groupe formé par les gommes arabiques, les acides gras saturés, linéaires ou ramifiés tels que l'acide palmitique, l'acide stéarique, l'acide myristique, l'acide laurique et leurs mélanges, les alcools gras saturés, linéaires ou ramifiés tels que l'alcool cétylique et l'alcool stéarylique.

Dans le cas où le véhicule d'application topique est un liquide, une crème, un gel ou une pommade, les tensioactifs sont ceux habituellement utilisés dans les formulations cosmétiques en lotion, en lait, en shampoing, en émulsion en crème, en gel ou en pommade. Ce sont des tensioactifs anioniques, cationiques ou zwitterioniques en fonction du véhicule d'application topique choisi. Ils sont choisis dans le groupe formé par les esters de glycol, les esters de polyoxyéthylèneglycol, les esters de sorbitane, les éthers d'alcool gras, les lipo-amino-acides, les sulfonamides, les ammoniums quaternaires, les bétaïnes.

Les agents gélifiants sont ceux couramment utilisés dans les formulations cosmétiques de gel. Ils sont choisis dans le groupe formé par l'hydroxyéthylcellulose, l'hydroxypropylcellulose, la méthylcellulose, la carboxyméthylcellulose, l'agar, les pectines, les alginates, les carraghénanes, la gélatine, ou encore les polysaccharides et les polymères carboxyvinyliques.

Les solvants miscibles à l'eau ou au moins en partie miscibles à l'eau sont des polyols tels que la glycérine, les glycols ou les polyéthylèneglycols ou leurs mélanges.

Les gaz propulseurs sont choisis parmi le propane, le butane, l'isobutane ou encore le diméthyléther.

Les différents constituants de ladite composition sont mélangés entre eux par simple agitation, par exemple dans un mélangeur, à la température ambiante, jusqu'à homogénéisation.

Encore un autre objet de la présente invention consiste en un dispositif monobloc ayant les caractéristiques susmentionnées, pour induire par voie topique, un soulagement des douleurs musculaires et/ou articulaires d'un sujet animal dans la zone de peau à traiter, notamment pour soulager les douleurs d'arthrose.

Selon un mode d'utilisation de la composition selon l'invention, dans le cas où le véhicule d'application topique est une matrice polymère et où le sujet à traiter est un animal, ladite matrice de la composition est maintenue par un lien contre la peau du sujet animal à traiter pendant un temps suffisant pour qu'un soulagement se manifeste. Le dispositif est efficace pendant une longue période allant de plusieurs jours à huit semaines et peut être porté sans interruption.

Afin de confirmer qu'un dérivé de l'acide salicylique, et notamment le salicylate de méthyle, est entrainé par l'huile essentielle auxiliaire pour traverser la peau, un test de perméation a été réalisé. Pour ce faire, on se réfère aux protocoles décrits dans la pharmacopée européenne (01/2008 :1011) et dans la littérature (Jacobi U., et al. Skin research and technology. 2007, 3) pour les dispositifs de diffusion transdermique. La température de travail est fixée à 32°C.

Selon l'invention, on met en œuvre la composition déposée sur la surface extérieure d'une membrane elle-même en contact par sa face intérieure avec le milieu récepteur. Il a été choisi d'utiliser la peau d'une oreille de cochon comme membrane et un mélange Eau/Ethanol (50/50) comme milieu récepteur. Le milieu récepteur doit être en contact avec la peau et l'on assure qu'il n'y a pas bulles d'air présentes.

Le milieu récepteur est ensuite prélevé à des temps précis pour analyse par chromatographie gazeuse. Les mélanges obtenus sont comparés à une solution témoin contenant le 1-8-cinéole comme marqueur de l'huile essentielle de romarin et le salicylate de méthyle comme marqueur de l'huile essentielle de gaulthérie couchée.

L'exemple ci-dessous est fourni à titre indicatif pour illustrer l'invention sans en restreindre sa portée.

Exemple : Dispositif monobloc avec une composition comportant un agent actif constitué d'huile essentielle de gaulthérie couchée et de romarin officinal pour soulager les douleurs articulaires chez le chien.

On dispose des intrants suivants :
- de l'huile essentielle de gaulthérie couchée commercialisée par NATURARUM. Cette huile essentielle contient entre 98 et 100% en poids de salicylate de méthyle.
- de l'huile essentielle de romarin officinal commercialisée par INTERAXION. Cette huile essentielle contient entre 50 et 55% en poids de 1-8-cinéole.
- de l'huile de coprah raffinée commercialisée par INTERAXION,
- des granulés de copolymères d'éthylène et d'acétate de vinyle (EVA) commercialisés par GAZECHIM sous la marque EVA ALCUDIA PA-538 qui contient 18% en poids d'acétate de vinyle. La température de transition vitreuse de ce polymère est donnée à 68°C.
- du colorant chocolat sous forme de granulés commercialisé par ELIAN sous la marque P4625C.

On dispose des matériels suivants :
- un mélangeur cylindrique de la marque PAPPENMEIER d'une capacité de 8 litres muni et d'un agitateur actionné par un moteur à variation de vitesse,
- une presse à injection de la marque SANDRETTO de série 8, développant une capacité de pression de 150 tonnes,
- le moule en acier monté sur ladite presse possède quatre empreintes en forme de collier d'une longueur de 75 cm.

Le diagramme de température du fourreau, de la trémie à la buse, est le suivant : 115°C (zone 1), 125°C (zone 2), 130°C (zone 3), 135°C (zone 4) avec un temps de maintien de 3 secondes à 65 bars et une température du moule à 155°C.
- un appareil chromatographie en phase gazeuse de la marque VARIAN, muni d'une colonne en polyéthylène glycol 20000 Da, type Optima-Wax 0.25m - 30m x 0.32 mm ID.

### Protocole :

### a- Préparation de l'agent actif de la composition :

Dans un bêcher de 250 mL, on introduit, à la température ambiante, successivement 74g d'huile essentielle de gaulthérie couchée et 26g d'huile essentielle de romarin. On rajoute 12g d'huile de coprah raffinée afin de mieux disperser les deux huiles essentielles. On agite faiblement à l'aide d'un barreau aimanté afin d'obtenir un mélange homogène qui constitue la composition liquide.

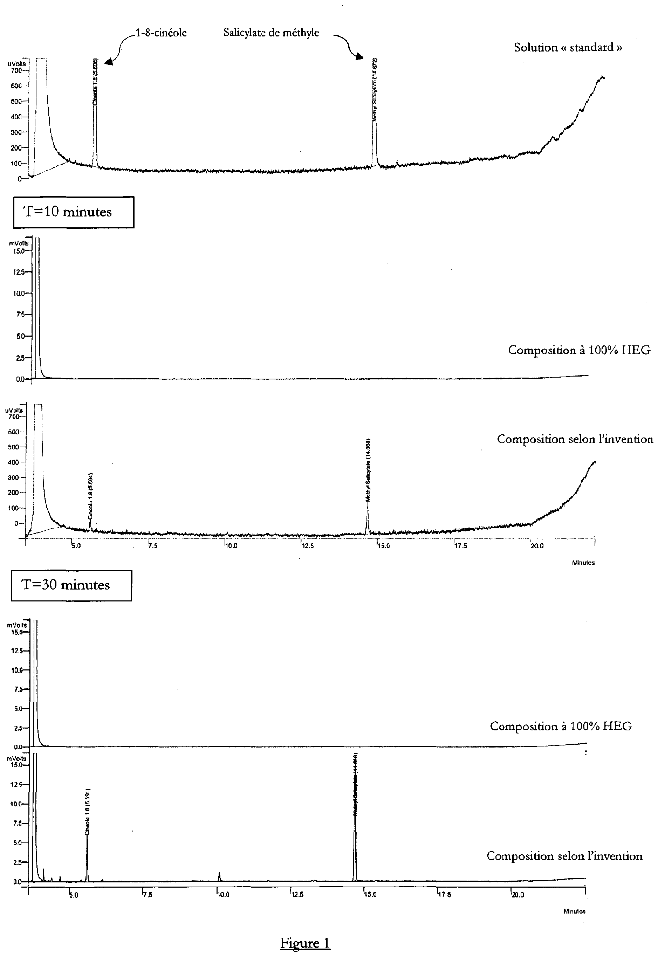

On pèse 135mg de la composition obtenue ci-dessus que l'on applique directement sur la peau d'une oreille de cochon sur une surface de 7,1cm², selon le protocole décrit précédemment. Le volume du milieu récepteur Eau/Ethanol (50/50) est de 140mL. Des prélèvements du milieu récepteur sont réalisés à 10 minutes et 30 minutes. Le chromatogramme obtenu est représenté par la figure 1.

Figure 1 : chromatogramme de la composition comprenant l'agent actif conforme à l'invention, déposée à la surface de peau d'oreille de cochon.

On constate qu'en présence de l'huile essentielle de romarin officinal (HER), la vitesse du passage de l'huile essentielle de gaulthérie couchée (HEG) est augmentée. Ce résultat confirme que l'huile essentielle de romarin officinal entraîne le passage transcutané du salicylate de méthyle. En revanche, lorsque la composition comporte un agent actif constitué uniquement de l'huile essentielle de gaulthérie, le salicylate de méthyle ne parvient pas à traverser la peau même au bout de 30 minutes.

### b- Incorporation de la composition dans l'EVA :

On préchauffe le réacteur à 70°C dans un bain d'huile. On y introduit, sous faible agitation, 830g de granulés d'EVA jusqu'à ce la température mesurée au sein desdits granulés avoisine 70°C. Ensuite, on introduit dans le réacteur, toujours sous faible agitation, la composition liquide obtenue précédemment. On laisse agiter jusqu'à ce que tout le liquide soit entièrement absorbé par le polymère puis on baisse la température à 25°C. A la fin de l'incorporation, les granulés d'EVA sont secs et légèrement gonflés. Enfin, on introduit 20g de colorant marron, toujours sous faible agitation. On vidange le réacteur, le compound ainsi obtenu est stocké dans un emballage hermétique à l'air et à l'humidité. Les granulés d'EVA obtenus à l'issue de cette étape contiennent 10% en poids d'huile essentielle de gaulthérie couchée (HEG) et 3,5% en poids d'huile essentielle de romarin officinal (HER).

### c- Mise en forme du compound en collier

On injecte le compound obtenu à l'étape b) du protocole en colliers pesant environ 41g en sortie de buse pour une longueur de 75 cm.

### Evaluation de la vitesse du passage transdermique du salicylate de méthyle

Pour ce faire, la masse du collier est déterminée de manière à respecter les conditions de manipulation « sink » à savoir que le taux maximum d'actif relargué dans le milieu doit être inférieur à 10% de la concentration saturante de l'actif dans ce milieu. Le milieu récepteur est prélevé puis analysés par chromatographie.

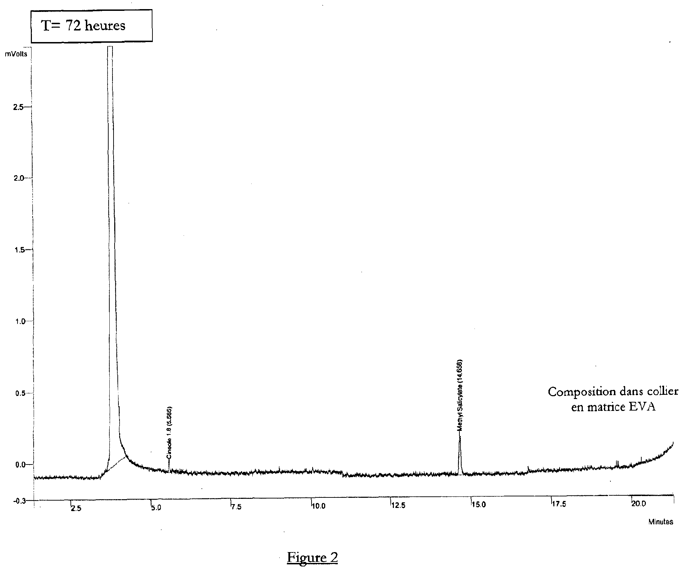

On pèse 1g de collier obtenu à l'étape c) du protocole ayant une surface de 3,1cm², selon le protocole décrit précédemment. Le volume du milieu récepteur Eau/Ethanol (50/50) est de 140mL. L'expérience est conduite pendant 72 heures puis 2mL du milieu récepteur sont prélevés et analysés par chromatographie en phase gazeuse. Les mélanges obtenus sont comparés à une solution « standard » contenant le 1-8-cinéole comme marqueur de l'huile essentielle de romarin officinal et le salicylate de méthyle comme marqueur de l'huile essentielle de gaulthérie couchée. Les résultats sont représentés par la figure 2.

Figure 2 : chromatogramme de la composition conforme à l'invention dont le véhicule d'application topique est un collier en matrice polymère d'EVA contenant la composition.

On constate que le salicylate de méthyle a traversé la peau depuis le collier jusque dans le milieu récepteur car les deux marqueurs y sont détectés. Cela montre d'une part que la matrice en polymère est capable de véhiculer l'agent actif pour que ce dernier aille sur la peau, et d'autre part que l'huile essentielle de romarin officinal entraine bien le passage du salicylate de méthyle à travers la peau d'oreille de cochon.

Le tableau 1 ci-dessous consigne les vitesses de transport du salicylate de méthyle (MeSa) de l'agent actif en fonction du véhicule d'application.

**Tableau 1 : Récapitulatif de la vitesse de transport du salicylate de méthyle à travers la peau**

| Essai | Agent actif | Véhicule | Vitesse (µg/mL/heure) |
|---|---|---|---|
| 1 | MeSa + huile essentielle romarin | Collier en EVA | 0,232 |
| 2 | MeSa + huile essentielle romarin | Collier en EVA | 0,102 |
| 3 | MeSa + huile essentielle romarin | Collier en EVA | 0,269 |
| 4 | MeSa + huile essentielle romarin | Huile de coprah | 327,4 |
| 5 | MeSa + huile essentielle citronnelle | Huile de coprah | 383,7 |
| 6 | MeSa + huile essentielle menthe poivrée | Huile de coprah | 257,7 |

### Tests d'efficacité effectués sur des chiens souffrant de douleurs articulaires et musculaires

L'efficacité du collier obtenu à l'étape c) du protocole a été évaluée sur un panel de quatre chiens sélectionnés dans un chenil parmi ceux âgés d'au moins 8 ans présentant des signes apparents de mobilité réduite caractérisés notamment par des difficultés à se lever et à se déplacer. Chacun des quatre chiens porte, autour de leur cou, un collier dont le ratio « composition/poids de l'animal » a été préalablement déterminé par la taille du collier.

Les animaux ont été mis en observation tous les jours pendant six semaines sans rien changer ni au niveau de leur alimentation, ni au niveau de leur rythme de vie.

Dès 48 heures, le plus handicapé des quatre s'est montré plus alerte et plus réactif vis-à-vis de ses congénères et du propriétaire du chenil, de plus, il a plus de facilité de faire des mouvements.

Après quatre jours de port, les quatre chiens ont montré une motricité significativement améliorée qui s'est traduite par des stations debout prolongées et davantage de promenades spontanées en particulier à l'extérieur. Le collier a donc amélioré la « vitalité » des chiens. Cet effet « vitalité » a été observé pendant six semaines sur le chien le plus handicapé.

## Revendications

1. Dispositif monobloc à action thérapeutique, qui comporte un véhicule d'application topique en matrice polymère et un agent actif induisant un soulagement de douleurs musculaires et/ou articulaires d'un sujet humain ou animal, ledit agent actif comportant du salicylate de méthyle apporté par l'huile essentielle de gaulthérie couchée et au moins une huile essentielle auxiliaire, ledit dispositif étant un collier, un bracelet ou un patch, **caractérisé en ce que** l'agent actif est incorporé dans ladite matrice polymère, qui représente de 80 à 90% en poids du dispositif, et que ledit agent actif comporte 52 à 85% en poids de salicylate de méthyle, la vitesse de transport du salicylate de méthyle à travers la peau étant augmentée grâce à au moins une huile essentielle auxiliaire choisi parmi l'huile essentielle de romarin officinal ou l'huile essentielle de citronnelle de Ceylan.

2. Dispositif monobloc à action thérapeutique, qui comporte un véhicule d'application topique en matrice polymère et un agent actif induisant un soulagement de douleurs musculaires et/ou articulaires d'un sujet humain ou animal, ledit agent actif comportant du salicylate de méthyle apporté par l'huile essentielle de gaulthérie couchée et au moins une huile essentielle auxiliaire, ledit dispositif étant un collier ou un bracelet, **caractérisé en ce que** l'agent actif est incorporé dans ladite matrice polymère, qui représente de 80 à 90% en poids du dispositif, et que ledit agent actif comporte 52 à 85% en poids de salicylate de méthyle, la vitesse de transport du salicylate de méthyle à travers la peau étant augmentée grâce à l'huile essentielle auxiliaire de menthe poivrée.

3. Dispositif selon la revendication 1 ou 2, **caractérisé en ce que** la (ou les) huile(s) essentielle(s) auxiliaire(s) de l'agent actif constitue(nt) entre 15 et 48% en poids dudit agent actif.

4. Dispositif selon l'une des revendications 1 à 3, **caractérisé en ce que** la matrice polymère est un copolymère d'éthylène et d'acétate de vinyle, dont le taux d'acétate de vinyle est compris entre 15 et 60% en poids.

5. Dispositif selon l'une des revendications 1 à 3, **caractérisé en ce que** la matrice polymère est un polymère thermoplastique choisi dans le groupe formé par les polyéthers block amides, les élastomères de polyuréthane thermoplastiques à base d'esters ou d'éthers, les copolyamides et les polyamides de grades absorbants, les polyéthylènes greffés amidon, les polychlorures de vinyle.

6. Dispositif selon l'une des revendications 1 à 3, **caractérisé en ce que** la matrice polymère est un polyuréthane réticulé à base d'isocyanate et de polyols ou une polyurée à base d'isocyanate et de polyamine.

7. Dispositif selon l'une des revendications 1, 3 à 6, **caractérisé en ce que** la (ou les) huile(s) essentielle(s) auxiliaire(s) comporte(nt) au moins un alcool monoterpénique choisi dans le groupe formé par le 1-8-cinéole, le géraniol et le linalol.

8. Dispositif selon l'une des revendications 2 à 6, **caractérisé en ce que** l'huile essentielle auxiliaire comporte du menthol.

9. Dispositif selon l'une des revendications 1 à 8, **caractérisé en ce que** l'huile essentielle de gaulthérie couchée comporte du salicylate de méthyle et constitue entre 1 et 15% en poids de l'ensemble constitué de toutes les huiles essentielles de la composition.

10. Dispositif selon l'une des revendications 1 à 8, **caractérisé en ce que** l'(es) huile(s) essentielle(s) auxiliaire(s) constitue(nt) de 0,15 à 9% en poids par rapport au poids total de l'ensemble constitué de toutes les huiles essentielles de la composition.

11. Dispositif selon l'une des revendications 1 à 10, **caractérisé en ce que** l'agent actif comporte au moins un additif de formulation choisi dans le groupe formé par des huiles végétales, des charges inertes d'origine minérale notamment sous forme de poudres, des colorants, des tensioactifs, du parfum de synthèse ou d'origine naturelle, ou leurs mélanges.

12. Dispositif selon l'une des revendications 1 à 11, pour induire, par voie topique, un soulagement des douleurs d'arthrose, **caractérisé en ce que** la matrice polymère, qui constitue le véhicule d'application de l'agent actif est maintenue par un lien contre la peau du sujet humain ou animal à traiter pendant un temps suffisant pour qu'un soulagement se manifeste.

## Patentansprüche

1. Einteilige Vorrichtung mit therapeutischer Wirkung, die ein Medium für eine topische Anwendung in einer Polymermatrix und einen Wirkstoff aufweist, der eine Linderung von Muskel- und/oder Gelenkschmerzen bei einem menschlichen oder tierischen Subjekt hervorruft, wobei der Wirkstoff Methylsalicylat aufweist, das durch das ätherische Öl von Wintergrün und mindestens ein ergänzendes ätherisches Öl eingebracht wird, wobei die Vorrichtung eine Halskette, ein Armband oder ein Pflaster ist, **dadurch gekennzeichnet, dass** der Wirkstoff in die Polymermatrix eingemischt ist, die zu 80 bis 90 Gew.-% die Vorrichtung darstellt, und dass der Wirkstoff zu 52 bis 85 Gew.-% Methylsalicylat aufweist, wobei die Transportgeschwindigkeit von Methylsalicylat durch die Haut dank mindestens einem ergänzenden ätherischen Öl erhöht ist, das aus ätherischem Öl von Rosmarinus officinalis oder ätherischem Öl von Zitronellgras aus Ceylon ausgewählt ist.

2. Einteilige Vorrichtung mit therapeutischer Wirkung, die ein Medium für die topische Anwendung in der Polymermatrix und einen Wirkstoff aufweist, der eine Linderung von Muskel- und/oder Gelenkschmerzen bei einem menschlichen oder tierischen Subjekt hervorruft, wobei der Wirkstoff Methylsalicylat aufweist, das durch das ätherische Öl von Wintergrün und mindestens ein ergänzendes ätherisches Öl eingebracht wird, wobei die Vorrichtung eine Halskette oder ein Armband ist, **dadurch gekennzeichnet, dass** der Wirkstoff in die Polymermatrix eingemischt ist, die zu 80 bis 90 Gew.-% die Vorrichtung darstellt, und dass der Wirkstoff zu 52 bis 85 Gew.-% Methylsalicylat aufweist, wobei die Transportgeschwindigkeit von Methylsalicylat durch die Haut dank des ätherischen Öls von Pfefferminze erhöht ist.

3. Vorrichtung nach Anspruch 1 bis 2, **dadurch gekennzeichnet, dass** das (oder die) ergänzende(n) ätherische(n) Öl(e) des Wirkstoffs zu zwischen 15 und 48 Gew.-% den Wirkstoff ausmacht/ausmachen.

4. Vorrichtung nach einem der Ansprüche 1 bis 3, **dadurch gekennzeichnet, dass** die Polymermatrix ein Copolymer aus Ethylen und Vinylacetat ist, dessen Vinylacetatgehalt zwischen 15 und 60 Gew.-% liegt.

5. Vorrichtung nach einem der Ansprüche 1 bis 3, **dadurch gekennzeichnet, dass** die Polymermatrix ein Thermoplast ist, der aus der Gruppe ausgewählt ist, bestehend aus Polyetherblockamiden, thermoplastischen Polyurethanelastomeren auf der Basis von Estern oder Ethern, Copolyamiden und Polyamiden von Absorptionsgrad, starken Pfropfpolyethylenen, Polyvinylchloriden.

6. Vorrichtung nach einem der Ansprüche 1 bis 3, **dadurch gekennzeichnet, dass** die Polymermatrix ein vernetztes Polyurethan auf der Basis von Isocyanat und Polyolen oder ein Polyharnstoff auf der Basis von Isocyanat und Polyamin ist.

7. Vorrichtung nach einem der Ansprüche 1, 3 bis 6, **dadurch gekennzeichnet, dass** das (oder die) ergänzende(n) ätherische(n) Öl(e) mindestens einen Monoterpenalkohol aufweist/aufweisen, der aus der Gruppe ausgewählt ist, bestehend aus 1-8-Cineol, Geraniol und Linalool.

8. Vorrichtung nach einem der Ansprüche 2 bis 6, **dadurch gekennzeichnet, dass** das ergänzende ätherische Öl Menthol aufweist.

9. Vorrichtung nach einem der Ansprüche 1 bis 8, **dadurch gekennzeichnet, dass** das ätherische Öl von Wintergrün Methylsalicylat aufweist und zu zwischen 1 und 15 Gew.-% die Gesamtheit ausmacht, die aus allen ätherischen Öle der Zusammensetzung besteht.

10. Vorrichtung nach einem der Ansprüche 1 bis 8, **dadurch gekennzeichnet, dass** das/die ergänzende(n) ätherische(n) Öl(e) zu 0,15 bis 9 Gew.-%, bezogen auf das Gesamtgewicht der Gesamtheit ausmacht/ausmachen, die aus allen ätherischen Öle der Zusammensetzung besteht.

11. Vorrichtung nach einem der Ansprüche 1 bis 10, **dadurch gekennzeichnet, dass** der Wirkstoff mindestens einen Formulierungszusatzstoff aufweist, der aus der Gruppe ausgewählt ist, bestehend aus Pflanzenölen, Inert-Füllstoffen mineralischen Ursprungs, insbesondere in Pulverform, Farbstoffen, Tensiden, synthetischem oder natürlichem Duft oder Mischungen davon.

12. Vorrichtung nach einem der Ansprüche 1 bis 11, zum Hervorrufen, auf topischem Wege, einer Linderung von Arthroseschmerzen, **dadurch gekennzeichnet, dass** die Polymermatrix, die das Medium für die Anwendung des Wirkstoffs ausmacht, während einer Zeit, die ausreicht, damit eine Linderung eintritt, durch eine Bindung an die Haut des zu behandelnden menschlichen oder tierischen Subjekts gehalten wird.

## Claims

1. Monobloc device for therapeutic action comprising a polymer matrix topical application carrier and an active agent for inducing relieving muscle and/or joint pain in an human or animal subject, said active agent comprises methyl salicylate brought in by wintergreen essential oil and at least one auxiliary essential oil, the said devices being a collar, bracelet or patch, wherein the polymer matrix, in which the active agent is incorporated, represents 80 to 90% by weight of the device, and the active agent comprises 52 to 85% by weight of methyl salicylate, the rate of transport of the methyl salicylate through the skin being increased by virtue of at least one essential oil chosen from rosemary essential oil (*Rosmarinus officinalis*) or Ceylan citronella essential oil.

2. Monobloc device for therapeutic action comprising a polymer matrix topical application carrier and an active agent for inducing relieving muscle and/or joint pain in an human or animal subject, said active agent comprises methyl salicylate brought in by wintergreen essential oil and at least one auxiliary essential oil, the said devices being a collar, bracelet or patch, wherein the polymer matrix, in which the active agent is incorporated, represents 80 to 90% by weight of the device, and the active agent comprises 52 to 85% by weight of methyl salicylate, the rate of transport of the methyl salicylate through the skin being increased by virtue of essential peppermint oil (*Mentha piperita*)*.*

3. Device according to claim 1 or 2, wherein the auxiliary essential oil of the active agent constitutes between 15 and 48% by weight of the active agent.

4. Device according to any one of claims 1 to 3, wherein the polymer matrix is a copolymer of ethylene and vinyl acetate (EVA), whose vinyl acetate content is between 15 and 60% by weight.

5. Device according to any one of claims 1 to 3, wherein the polymer matrix is a thermoplastic polymer formed by polyether block amides, ester- or ether-based thermoplastic polyurethane elastomers, absorbent-grade copolyamides and polyamides, starch-grafted polyethylenes and vinyl polychlorides.

6. Device according to any one of claims 1 to 3, wherein the polymer matrix is an isocyanateand polyol-based cross-linked polyurethane or an isocyanate- and polyamine-based polyuria.

7. Device according to any one of claim 1, 3 to 6, wherein the auxiliary essential oil contain at least one monoterpene alcohol chosen from the group formed by 1,8-cineole, geraniol and linalool.

8. Device according to any one of claim 1, 3 to 6, wherein the auxiliary essential oil contains menthol.

9. Device according to any one of claims 2 to 8, wherein the essential wintergreen oil contains methyl salicylate and constitutes between 1 and 15% by weight in relation to the total weight of the assembly consisting of all of the essential oils of the composition.

10. Device according to any one of claims 1 to 9, wherein the auxiliary essential oil constitutes from 0.15 to 9% by weight in relation to the total weight of the assembly consisting of all of the essential oils of the composition.

11. Device according to any one of claims 1 to 10, wherein the active agent contains at least one formulation additive formed by vegetable oils, inert loads of mineral origin particularly in the form of powders, dyes, surfactants, synthetic or natural perfume, or a mixture thereof.

12. Device according to any one of claims 1 to 11, to topically induce arthritic pain relief wherein the polymer matrix that constitutes the application carrier of the active agent is held by a bond against the skin of the human or animal subject to be treated for a sufficient time for relief to manifest.
